# EUROPÄISCHE PATENTSCHRIFT

(11) **EP 1 212 191 B1**
(45) Veröffentlichungstag und Bekanntmachung des Hinweises auf die Patenterteilung: **25.02.2004**
(21) Anmeldenummer: 00974283.4
(22) Anmeldetag: 04.09.2000
(51) Int. Cl.: B30B 11/34, B30B 11/08

(54) **VERFAHREN UND VORRICHTUNG ZUR HERSTELLUNG VON TABLETTEN**
METHOD AND DEVICE FOR PRODUCING TABLETS
PROCEDE ET DISPOSITIF DE PRODUCTION DE COMPRIMES

(30) Priorität: 02.09.1999 DE 19941997
(43) Veröffentlichungstag der Anmeldung: 12.06.2002
(73) Patentinhaber: Voss, Gunter Meinhardt, D-86911 Diessen (DE)
(72) Erfinder: Voss, Gunter Meinhardt, D-86911 Diessen (DE)
(74) Vertreter: Rösler, Uwe, Dipl.-Phys.
(86) Internationale Anmeldenummer: PCT/DE2000/003029
(87) Internationale Veröffentlichungsnummer: WO 2001/015889

(56) Entgegenhaltungen:
- DE-A- 4 437 442
- DE-C- 287 776
- DE-U- 29 807 840
- US-A- 2 800 087
- US-A- 3 255 279
- US-A- 3 682 742
- US-A- 4 139 589
- US-A- 4 460 490
- US-A- 4 601 866

## Beschreibung

### Technisches Gebiet

Die Erfindung bezieht sich auf Verfahren zur Herstellung einer Tablette oder dgl. aus wenigstens einem Mantelmaterial und wenigstens einem Kernmaterial, das insbesondere aus viskosem Material bestehen kann, unter Verwendung einer Tablettenpresse mit einem Oberstempel und einem vorzugsweise ebenfalls (teilweise) beweglichen Unterstempel.

Ferner bezieht sich die Erfindung auf eine Vorrichtung zur Herstellung von Tabletten, die insbesondere zur Durchführung des erfindungsgemäßen Verfahrens geeignet ist.

Derartige Verfahren und Vorrichtungen sind zur Herstellung von beliebigen Tabletten, wie beispielsweise Tabletten für pharmazeutische Anwendungen oder für Anwendungen im Lebensmittel- bzw. Nahrungsergänzungsbereich, wie Bonbons, Vitamindrops usw., oder sonstigen "tablettenähnlichen" Gegenständen, wie z.B. von sogenannten Reinigungs-TAB's für Waschmaschinen oder Geschirrspülmaschinen oder z.B. Tabletten zur Schädlingsbekämpfung, für technische Anwendungen etc. einsetzbar.

### Stand der Technik

Ein Verfahren, von dem bei der Formulierung der Oberbegriffs des Patentanspruchs 1 ausgegangen wird, ist aus der EP 0 773 866 B1 bekannt. Auf diese Druckschrift und den in dieser Druckschrift genannten Stand der Technik wird im übrigen zur Erläuterung aller hier nicht näher beschriebenen Einzelheiten ausdrücklich Bezug genommen.

Aus dieser Druckschrift ist ein Verfahren zur Herstellung sogenannter "Manteltabletten" bekannt. Dieses bekannte Verfahren erlaubt die Herstellung von Tabletten, bei denen ein oder mehrere Tablettenkerne in ein Mantelmaterial bzw. ein Mantelgranulat eingebettet und/oder in die Oberfläche des Mantelmaterials eingesetzt sind.

Ähnliche Verfahren bzw. Tabletten sind auch aus der WO 99/06522, der WO 99/27063 oder der WO 99/27067 bekannt. Auch auf diese Druckschriften wird zur Erläuterung aller hier nicht näher beschriebenen Begriffe und Einzelheiten sowie zur Erläuterung der allgemeinen Anwendbarkeit der Erfindung ausdrücklich Bezug genommen.

Allen aus diesen Druckschriften bekannten Verfahren ist gemeinsam, daß zur Herstellung einer "formschönen" Tablette, die in ihrer Oberfläche wenigstens einen - insbesondere sichtbaren - Tablettenkern aufweist, mehrere Schritte erforderlich sind:

So ist es bei dem aus der EP 0 773 866 B1 bekannten Verfahren - sofern eine formschöne Tablette hergestellt werden soll - in der Regel erforderlich, zunächst Mantelgranulat in die sogenannte Matrize einzubringen, das Mantelgranulat derart zu pressen bzw. vorzupressen, daß es eine Vertiefung aufweist, und anschließend den Tablettenkern in die (vorgepreßte) Vertiefung einzubringen. Erfolgt die Herstellung nur in einem einzigen Preßschritt ist nicht bei allen Materialien gewährleistet, daß der Kern eine "saubere bzw. schöne", genau definierte Form und insbesondere eine "scharfe" Umrandung hat.

### Darstellung der Erfindung

Der Erfindung liegt die Aufgabe zugrunde, Verfahren sowie Vorrichtungen zur Herstellung von Tabletten anzugeben, die die Herstellung von Tabletten mit wenigstens einem in die Oberfläche eingebetteten Tablettenkern im Vergleich zum Stand der Technik vereinfachen.

Erfindungsgemäße Lösungen dieser Aufgabe sind in den nebengeordneten, auf ein Verfahren gerichteten Ansprüchen 1,2 und 3 angegeben. In den Ansprüchen 10 folgende ist eine Vorrichtung beschrieben, die sich insbesondere zur Durchführung der erfindungsgemäßen Verfahren eignet.

Bei dem im Anspruch 1 angegebenen Verfahren ist der Unterstempel der Tablettenpresse mit wenigstens einer Ausnehmung im Stempel selbst oder in wenigstens einem Vorsprung des Stempels versehen. Das oder die Kernmaterialien werden vor dem Einbringen des oder der Mantelmaterialien in die von dem Unterstempel und den Seitenwänden gebildete Matrize in die Ausnehmung eingebracht.

Damit ist es nur erforderlich, den oder die Tablettenkerne auf den bzw. die entsprechenden Bereiche des Unterstempels aufzubringen; dies kann beispielsweise mit jeweils einer Düse erfolgen, mit der der jeweilige Tablettenkern aufgespritzt wird. Anschließend wird die Matrize mit dem oder den Mantelmaterialien - gegebenenfalls mit geschichteten unterschiedlichen Mantelmaterialien - aufgefüllt-. Bei dem sich anschließenden Preßvorgang begrenzt der oder die Vorsprünge den Bereich, über den sich die Tablettenkerne "ausbreiten", so daß eine Tablette mit einem "formschönen" Kern hergestellt wird. Die Oberfläche des Kerns kann dabei insbesondere durch die Formgebung des Bodens des Stempels zwischen den Vorsprüngen beeinflußt werden.

Alternativ kann zunächst das Mantelmaterial in die vom Unterstempel und den Seitenwänden gebildete Matrize eingefüllt werden. Dabei können insbesondere unterschiedliche Mantelmaterialien geschichtet eingebracht werden. Anschließend wird auf die dem Oberstempel zugewandte Oberfläche des Mantelmaterials das-oder die Kernmaterialien aufgebracht, wobei diese beiden Schritte selbstverständlich auch vertauscht oder gleichzeitig ausgeführt werden können. Um eine "saubere" Verteilung des Kerns in der Oberfläche der Tablette zu erhalten, wird die Tablette mit einem Oberstempel gepreßt, der wenigstens eine Ausnehmung im Stempel selbst oder in wenigstens einem Vorsprung des Stempels aufweist, der die Verteilung des jeweiligen Kernmaterials bzw. Matelmaterials in der fertig gepreßten Tablette begrenzt. Werden mehrere Kerne verwendet, so ist es selbstverständlich bevorzugt, den Oberstempel mit mehreren Vorsprüngen zu versehen, die dem jeweiligen Aufbringort der Tablettenkerne angepaßt sind.

Der wenigstens eine in sich geschlossene Vorsprung kann beliebige Formen haben: beispielsweise kann er kreisrund sein oder die Form eines Logos (Firmenzeichen, Marke etc.) oder dgl. haben.

Besonders vorteilhaft ist es, als Tablettenpresse einen sogenannten Rundläufer bzw. eine Rotationspresse zu verwenden. Mit derartigen Tablettenpresse wird ein hoher Ausstoß, typischerweise mehrere 10.000 Tabletten pro Stunde erreicht.

Um bei derart hohen Produktionszahlen das oder die Kernmaterialien entsprechend einbringen zu können, ist es bevorzugt, wenn zum Dosieren des oder der Kernmaterialien jeweils mindestens ein Proportionalventil verwendet wird.

Das oder die Mantelmaterialien sowie das oder die Kernmaterialien sind selbstverständlich dem jeweiligen Einsatzfall der Tablette angepaßt. Als Kernmaterialien bzw. als Trägermaterialien für die in dem Kern vorhandene Substanzen können die verschiedensten Materialien, wie sie unter anderem in der EP 0 773 866 B1 angegeben sind, verwendet werden. Insbesondere können die Kernmaterialien, die in das oder die Mantelmaterialien eingebracht bzw. an dem oder den Mantelmaterialien angebracht werden, unterschiedliche Auf- und/oder Ablöseverhalten als Funktion der Zeit, der Temperatur, des pH-Wertes und/oder anderer Parameter aufweisen.

Wenn Kernmaterialien beispielsweise auf Paraffin-Basis, aber auch andere Materialien, wie beispielsweise Schmelzen verwendet werden, ist es weiterhin bevorzugt, wenn das im Kontakt mit dem (den) Kernmaterial(ien) stehende Werkzeug, d.h. der Unterstempel bzw. der Oberstempel gekühlt wird.

Hierzu kann daß der Ober- und/oder der Unterstempel bzw. der zweite Stempel eine Kühleinrichtung aufweisen, die insbesondere wenigstens einen Kanal aufweisen kann, durch den ein kaltes Fluid, wie beispielsweise ein übliches Kühlfluid, z.B. auf Frigen-Basis oder auch flüssiger Stickstoff strömt.

Weiterhin ist es möglich, daß nach dem Einfüllen des oder der Mantelmaterialien und vor dem Pressschritt die dem Oberstempel zugewandte Oberfläche formgebend bearbeitet wird. Dies kann mit einem geeigneten Werkzeug, wie beispielsweise einem Vorpreß-Stempel erfolgen.

Um ein Anhaften insbesondere von adhäsiven Kernmaterialien an dem jeweiligen Werkzeug zu verhindern, ist es weiterhin bevorzugt, wenn vor dem Aufbringen des oder der Kernmaterialien auf den Unterstempel bzw. nach dem Aufbringen des oder der Kernmaterialien auf das Mantelmaterial und vor dem Preßschritt zumindest auf das Kernmaterial eine Trennschicht aufgebracht wird.

Ferner ist es möglich, die einzelnen Schichten der Mantel- und/oder Kernmaterialien mit Schichten einzuhüllen, die die Materialien schützen, ein spezielles Auflösungsverhalten zeigen und/oder weitere Funktionssubstanzen enthalten.

Eine weitere Verfahrensalternative sieht den Einsatz eines Oberstempels mit wenigstens einem Vorsprung vor, der einen Bereich umschließt, in den das Kernmaterial eingebracht wird. Das Mantelmaterial wird hingegen in die vom Unterstempel und den Seitenwänden gebildete Matrize eingefüllt. Weitere Einzelheiten hierzu sind dem nachfolgenden Ausführungsbeispiel zu entnehmen

Eine Vorrichtung zur Durchführung der erfindungsgemäßen Verfahren ist im Anspruch 10 folgendermaßen beschrieben:

Die erfindungsgemäße Vorrichtung kann insbesondere eine an sich bekannte Tablettenpresse mit wenigstens einer Dosierstation für jedes Kernmaterial, mit wenigstens einer Matrize, deren Boden vorzugsweise zumindest teilweise von einem beweglichen Unterstempel gebildet wird, und mit einem Oberstempel aufweisen. Die Vorrichtung zeichnet sich dadurch aus, dass der Ober- und/oder der Unterstempel mit wenigstens einer Ausnehmung im Stempel selbst oder in wenigstens einem Vorsprung des Stempels versehen ist, welche beim Preßvorgang das Kernmaterial umschließt.

Jede Dosierstation kann über ein oder mehrere Ventile für jedes Kernmaterial verfügen. Die Ventile können bevorzugt so ausgebildet sein, daß sie im Bereich von typischerweise 100 gs eine einstellbare Menge des jeweiligen Kernmaterials ausstoßen.

Hierzu können die Ventile Propartionalventile, also Ventile mit einem einstellbaren Öffnungsquerschnitt oder Ventile sein, deren Öffnungsquerschnitt zwar fest ist, die aber bezüglich ihrer Öffnungszeit geeignet ansteuerbar sind, so daß die ausgetragene Menge des Kernmaterials über die Öffnungszeit des Ein/Aus-Ventils gesteuert bzw. geregelt wird.

Die Ventile können in an sich bekannter Weise aufgebaut und beispielsweise Piezoventile oder Pneumatik- und/ oder Hydraulikventile sein. in dem Pneumatik- und/oder Hydraulikkreis können wiederum elektrisch angesteuerte Piezoventile vorgesehen sein.
weiterhin kann die Dosierstation(en) das Ausbringen des oder der Kernmaterialien mit hohem Drücken erlauben, so
daß auch halb erstarrte Schmelzen auf den Unterstempel aufbringbar sind.

Ferner ist es möglich, daß die Ausnehmung Bestandteil eines zweiten Stempels ist, der in dem Oberstempel bzw. in dem Unterstempel beweglich angeordnet ist.

Wenn die Dosierstation eine von der Steuerung der Tablettenpresse unabhängige Steuereinheit aufweist, an der die Signale beispielsweise von Näherungssensoren anliegen, kann die Dosierstation an einer herkömmlichen Tablettenpresse nachgerüstet werden, ohne daß in die Steuerung der Tablettenpresse eingegriffen wird.

Als Steuereinheit können alle handelsüblichen Rechner oder Industriesteuerungen eingesetzt werden.

### Kurze Beschreibung der Zeichnung

Die Erfindung wird nachstehend anhand von Ausführungsbeispielen unter Bezugnahme auf die Zeichnungen näher beschrieben in der zeigen:
- Fig. 1: ein erstes Ausführungsbeispiel der Erfindung, bei dem das Kernmaterial auf den Oberstempel aufgebracht wird,
- Fig. 2: eine Ansicht und einen Schnitt durch eine mit einer Vorrichtung gemäß Fig. 1 hergestellte Tablette, und
- Fig. 3: ein zweites Ausführungsbeispiel der Erfindung, bei dem das Kernmaterial auf den Unterstempel aufgebracht wird.

### Beschreibung von Ausführungsbeispielen

In den folgenden Figuren bzw. Teilfiguren sind gleiche Teile immer mit denselben Bezugszeichen bezeichnet. Dabei wird zur Verbesserung der Übersichtlichkeit in einigen Teilfiguren auf Bezugszeichen verzichtet.

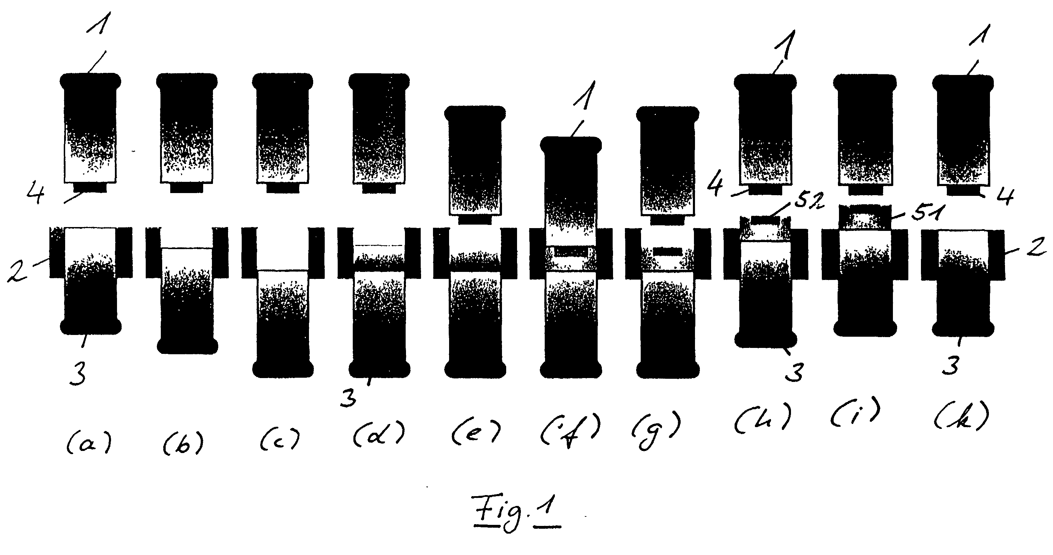

In Fig. 1 ist ein erstes Ausführungsbeispiel der Erfindung dargestellt, bei dem das Kernmaterial auf einen Oberstempel 1 aufgebracht wird.

Die Vorrichtung weist hierzu neben dem Oberstempel 1 eine Matrize auf, die von einer (in sich geschlossenen) Seitenwand 2 beispielsweise eines Rundläufertisches einer Tablettenpresse und einem beweglichen, d. h. verschiebbaren Unterstempel 3 dieser Tablettenpresse gebildet wird. Der Oberstempel 1 weist an seiner der Matrize zugewandten Unterseite einen Vorsprung 4 auf, der in sich geschlossen ist, so daß er einen bestimmten (inneren) Bereich der Fläche der Unterseite des Oberstempels von dem (äußeren) Rest der Unterseite abteilt. Eine in Fig. 1 nicht dargestellte Dosierstation bringt das Kernmaterial in den von dem Vorsprung 4 umschlossenen Bereich ein.

Im folgenden soll die Arbeitsweise der in Fig. 1 dargestellten Vorrichtung anhand der Teilbilder a bis k erläutert werden, die aufeinanderfolgende Phasen des Herstellvorgangs einer Tablette zeigen:

Teilbild a zeigt die Vorrichtung zu Beginn des Herstellvorgangs: der bewegliche Unterstempel 3 ist in der Matrize nach oben gefahren, der Oberstempel 1 mit dem Vorsprung 4 ist zurückgezogen. Zur Vorbereitung des Einfüllens des Mantelmaterials, das beispielsweise ein Granulat sein kann, wird der Unterstempel 2 zurückgezogen (Teilbild b).

Anschließend wird von der nicht dargestellten Dosierstation das Kernmaterial in den von dem Vorsprung 4 umschlossenen Bereich eingebracht (Teilbild c). Nunmehr wird in die von der Seitenwand 2 und dem Unterstempel 3 gebildete Matrize das Mantelmaterial eingebracht (Teilbild d). Die in den Teilbildern c und d dargestellten Schritte können selbstverständlich auch vertauscht oder gleichzeitig ausgeführt werden.

Im Teilbild e ist dargestellt, daß der Oberstempel 1 abgesenkt wird. Durch das weitere Absenken des Oberstempels 1 verpreßt dieser mit seiner Unterseite das Mantelmaterial und das auf seiner Unterseite innerhalb des Vorsprungs 4 befindliche Kernmaterial in einem einzigen Preßschritt (Teilbild f).

Anschließend wird der Oberstempel 1 zurückgezogen (Teilbild g). Die Tablette 5, bestehend aus gepreßtem Mantelmaterial 51 und einem in die dem Oberstempel 1 zugewandte Oberfläche der Tablette 5 eingelagertem Kern 52 ist fertig. Durch "Hochfahren" des Unterstempels 3 wird die Tablette 5 aus der Matrize ausgestoßen (Teilbildern h und i).

Nach dem Ausstoßen befindet sich die Vorrichtung wieder in ihrer Grundstellung (Teilbild k, das Teilbild a entspricht).

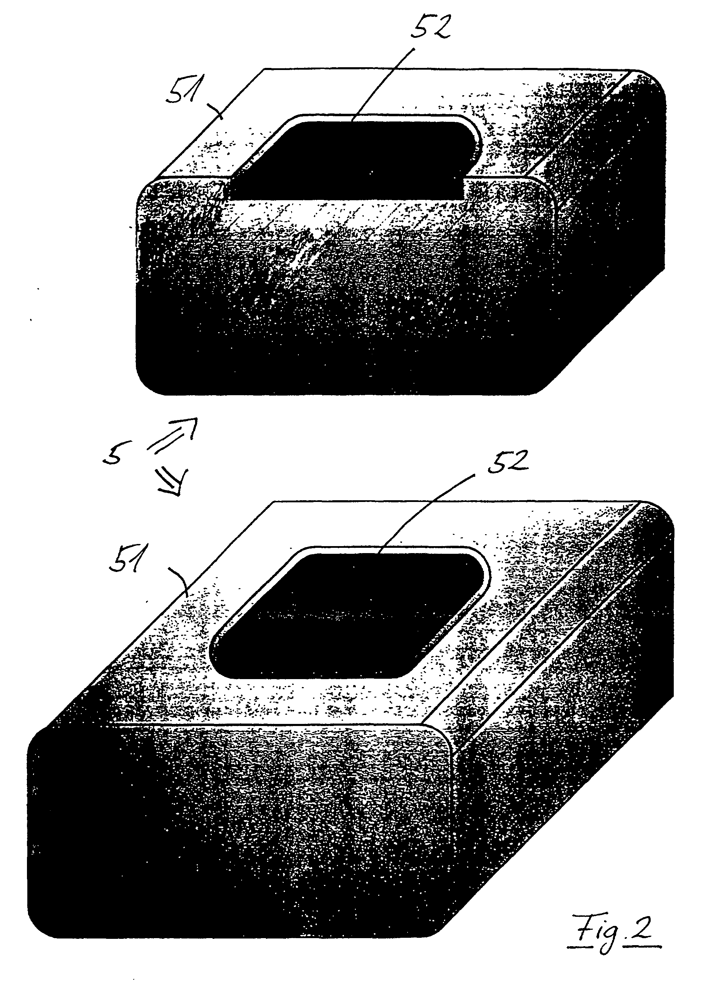

Fig. 2 zeigt die mit der in Fig. 1 dargestellten Vorrichtung hergestellte Tablette 5 in einer Ansicht (unteres Bild) und in einem Schnitt (oberes Bild). Durch den Vorsprung 4 entsteht in der Tablette ein entsprechender Rücksprung, der zu einer "sauberen" Begrenzungen des Kerns 52 gegenüber dem umgebenden Mantelmaterial 51 führt.

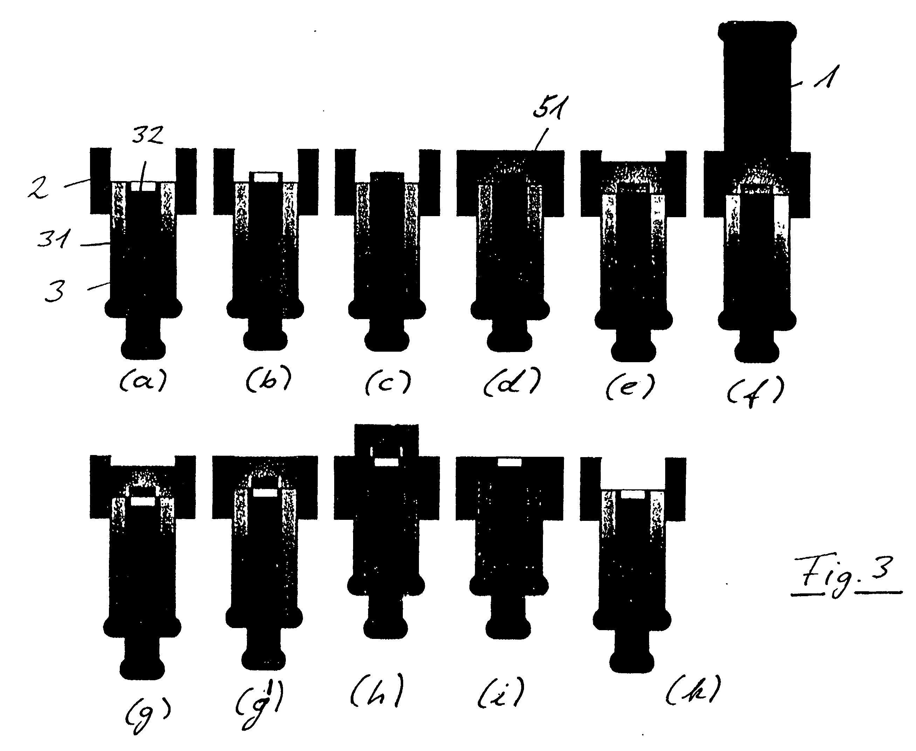

Fig. 3 zeigt ein zweites Ausführungsbeispiel der Erfindung, bei dem gleiche Teile wie in Fig. 1 mit den selben Bezugszeichen versehen sind.

Teilbild a zeigt die Vorrichtung in ihrer Ausgangsstellung, in der der Unterstempel 3 bereits zurückgezogen ist. Der Unterstempel 3 weist bei dem gezeigten Ausführungsbeispiel einen zweiten Stempel, nämlich einen Innenstempel 31 auf, in dessen Oberseite eine Ausnehmung 32 vorgesehen ist.

Zur Vorbereitung des Einbringens des Tablettenkerns 52 wird bei diesem Ausführungsbeispiel der Innenstempel 31 gegenüber dem Unterstempel 3 hochgefahren (Teilbild b). Anschließend wird mittels einer nicht gezeigten Dosierstation das Kernmaterial in die Ausnehmung 32 eingebracht (Teilbild c).

Teilbild d zeigt den Zustand, in dem die von dem Unterstempel 2, dem Innenstempel 3 und den Seitenwänden 2 gebildete Matrize mit Mantelmaterial 51, also beispielsweise einem Granulat aufgefüllt ist. Anschließend werden der Unterstempel 2 und der Innenstempel 3 zurückgezogen (Teilbild e).

Nun erfolgt der Preßvorgang, bei dem der Oberstempel 1 in die Matrize "eintaucht" (Teilbild f).

Nach Beendigung des Preßvorgangs wird der Innenstempel 31 zurückgezogen, so daß er nicht mehr "formschlüssig" in die Tablette 5 eingreift. Durch Hochfahren des Unterstempels 3 und des Innenstempels 31 erfolgt das Auswerfen der Tablette (Teilbildern g, g' und h).

Teilbild i zeigt die Vorrichtung nach der Beendigung des Auswerfens.

Teilbild k zeigt die Vorrichtung wiederum in der Grundstellung, die Teilbild a entspricht.

Vorstehend ist die Erfindung anhand von Ausführungsbeispielen beschrieben worden. Selbstverständlich sind innerhalb des allgemeinen, den Ansprüchen entnehmbaren Erfindungsgedankens die verschiedensten Abwandlungen möglich:

So ist es beispielsweise möglich, die Merkmale des ersten und des zweiten Ausführungsbeispiels miteinander zu kombinieren. Auch ist es möglich, Kerne in der Oberfläche mit in dem Inneren des Mantelmaterials eingelagerten Kernen in einer Tablette und in einem einzigen Herstellvorgang zu kombinieren.

Selbstverständlich können beliebige Steuereinheiten, wie PC's, Industriesteuerungen zur Ansteuerung der einzelnen Komponenten - Stempel, Dosierstationen etc. - und des gesamten Verfahrensablaufs eingesetzt werden.

In jedem Falle sind die erfindungsgemäßen Verfahren und Vorrichtungen geeignet, Tabletten oder allgemein gesagt - gegebenenfalls aus mehreren Schichten oder Materialien bestehende - Formlinge oder Komprimate mit beliebigen Kernen und gegebenenfalls Beschichtungen des Kernund/oder Mantelmaterials, wie Isolier- bzw. Trennschichten herzustellen. Die Beschichtungen können dabei auch Funktionssubstanzen etc. enthalten, wie dies z.B. in einer speziellen Ausführungsform in der EP-A-0 738 136 beschrieben ist, auf die im übrigen hinsichtlich des Aufbringens von Isolier- bzw. Trennschichten gegebenenfalls mit zusätzlichen Funktionssubstanzen ausdrücklich verwiesen wird.

## Patentansprüche

1. Verfahren zur Herstellung einer Tablette oder dgl. aus wenigstens einem Mantelmaterial (51) und wenigstens einem Kemmaterial (52), das insbesondere aus viskosem Material bestehen kann, unter Verwendung einer Tablettenpresse mit einem Oberstempel (1) und einem vorzugsweise ebenfalls beweglichen Unterstempel (3),
**dadurch gekennzeichnet, dass** der Unterstempel (3) mit wenigstens einer Ausnehmung im Stempel selbst oder in wenigstens einem Vorsprung (4) des Stempels versehen ist, und
dass das oder die Kernmaterialien (52) vor dem Einbringen des oder der Mantelmaterialien (51) in die von dem Unterstempel (3) und den Seitenwänden (2) gebildete Matrize in die Ausnehmung(en) eingebracht werden.

2. Verfahren zur Herstellung einer Tablette oder dgl. aus wenigstens einem Mantelmaterial (51) und wenigstens einem Kernmaterial, das insbesondere aus viskosem Material bestehen kann, unter Verwendung einer Tablettenpresse mit einem Oberstempel (1) und einem vorzugsweise ebenfalls beweglichen Unterstempel (3),
**dadurch gekennzeichnet, dass** zunächst das Mantelmaterial (51) in die vom Unterstempel (3) und den Seitenwänden (2) gebildete Matrize eingefüllt wird, und dass anschließend auf die dem Oberstempel (1) zugewandte Oberfläche des Mantelmaterials (51) das Kernmaterial (52) aufgebracht wird, und
dass dann die Tablette mit einem Oberstempel (1) gepreßt wird, der wenigstens eine Ausnehmung im Stempel selbst oder in wenigstens einem Vorsprung (4) des Stempels aufweist, der die Verteilung des jeweiligen Kernmaterials (52) bzw. Mantelmaterials (51) in der fertig gepreßten Tablette (5) begrenzt.

3. Verfahren zur Herstellung einer Tablette oder dgl. aus wenigstens einem Mantelmaterial (51) und wenigstens einem Kernmaterial (52), das insbesondere aus viskosem Material bestehen kann, unter Verwendung einer Tablettenpresse mit einem Oberstempel (1) und einem vorzugsweise ebenfalls beweglichen Unterstempel (3),
**dadurch gekennzeichnet, dass** der Oberstempel (3) wenigstens einen Vorsprung (4) vorsieht, der einen Bereich umschließt, in den das Kernmaterial eingebracht wird, und dass das Mantelmaterial (51) in die vom Unterstempel (3) und den Seitenwänden (2) gebildete Matrize eingefüllt wird.

4. Verfahren nach einem der Ansprüche 1 bis 3,
**dadurch gekennzeichnet, daß** bei Verwendung einer Schmelze für das oder die Kernmaterialien (52) das im Kontakt mit dem (den) Kernmaterial(ien) stehende Werkzeug, d.h. der Unterstempel (3) bzw. der Oberstempel (1) gekühlt wird.

5. Verfahren nach einem der Ansprüche 1 bis 4,
**dadurch gekennzeichnet, daß** nach dem Einfüllen des oder der Mantelmaterialien (51) in die Matrize der Preßschritt erfolgt.

6. Verfahren nach einem der Ansprüche 1 bis 5,
**dadurch gekennzeichnet, daß** nach dem Einfüllen des oder der Mantelmaterialien (51) und vor dem Preßschritt die dem Oberstempel (1) zugewandte Oberfläche formgebend bearbeitet wird.

7. Verfahren nach einem der Ansprüche 1, 3 bis 6,
**dadurch gekennzeichnet, daß** vor dem Aufbringen des oder der Kernmaterialien (52) auf den Unterstempel (3) eine Trennschicht aufgebracht wird.

8. Verfahren nach einem der Ansprüche 2, 4 bis 7,
**dadurch gekennzeichnet, daß** nach dem Aufbringen des oder der Kernmaterialien (52) auf das Mantelmaterial (51) und vor dem Preßschritt zumindest auf das Kernmaterial (52) eine Trennschicht aufgebracht wird.

9. Verfahren nach einem der Ansprüche 1 bis 8,
**dadurch gekennzeichnet, daß** wenigstens eines der Kernmaterialien (52) ein adhäsives Material ist.

10. Vorrichtung zur Herstellung von Tabletten oder dgl. aus wenigstens einem Mantelmaterial (51) und wenigstens einem Kernmaterial (52) insbesondere zur Durchführung des Verfahrens nach einem der Ansprüche 1 bis 9, mit
- wenigstens einer Matrize, deren Boden vorzugsweise zumindest teilweise von einem beweglichen Unterstempel (3) gebildet wird,
- einem Oberstempel (1) sowie
- wenigstens einer Dosierstation für jedes Kernmaterial (52),
**dadurch gekennzeichnet, dass** der Ober- (1) und/oder der Unterstempel (3) mit wenigstens einer Ausnehmung im Stempel selbst oder in wenigstens einem Vorsprung (4) des Stempels versehen ist, welche beim Preßvorgang das Kernmaterial (52) umschließt.

11. Vorrichtung nach Anspruch 10,
**dadurch gekennzeichnet, daß** die Dosierstation(en) das (die) Kernmaterial (52) (ien) in den von dem Vorsprung (4) umschlossenen Bereich bzw. in die Ausnehmung eindosiert (eindosieren).

12. Vorrichtung nach Anspruch 10 oder 11,
**dadurch gekennzeichnet, daß** die Dosierstation(en) das (die) Kernmaterial(ien) (52) in den Bereich der Oberfläche des oder der Mantelmaterialien (51) eindosieren, der dem bzw. den von dem Vorsprung (4) umschlossenen Bereich(en) bzw. der Ausnehmung(en) gegenüber liegt.

13. Vorrichtung nach einem der Ansprüche 10 bis 12,
**dadurch gekennzeichnet, daß** die Ausnehmung (4) Bestandteil eines zweiten Stempels ist, der in dem Oberstempel (1) bzw. in dem Unterstempel (3) beweglich angeordnet ist.

14. Vorrichtung nach einem der Ansprüche 10 bis 13,
**dadurch gekennzeichnet, daß** der Ober- (1) und/oder der Unterstempel (3) bzw. der zweite Stempel eine Kühleinrichtung aufweisen.

15. Vorrichtung nach Anspruch 14,
**dadurch gekennzeichnet, daß** die Kühleinrichtung wenigstens einen Kanal aufweist, durch den ein kaltes Fluid strömt.

16. Vorrichtung nach einem der Ansprüche 10 bis 15,
**dadurch gekennzeichnet, daß** die Dosierstation ein oder mehrere Ventile für jedes Kernmaterial aufweist.

17. Vorrichtung nach Anspruches 16,
**dadurch gekennzeichnet, daß** das oder die Ventile derart ausgebildet sind, daß sie in einem Zeitbereich von 100 µs proportional hinsichtlich der freigegebenen Öffnung oder bezüglich der Länge ihrer öffnungszeit ansteuerbar sind.

18. Vorrichtung nach einem der Ansprüche 10 bis 17,
**dadurch gekennzeichnet, daß** die Dosierstation(en) das Ausbringen des oder der Kernmaterialien (52) mit hohem Drücken erlauben, so daß auch halb erstarrte Schmelzen auf den Ober- (1) und/oder den Unterstempel (3) aufbringbar sind.

19. Vorrichtung nach einem der Ansprüche 10 bis 18,
**dadurch gekennzeichnet, daß** der wenigstens eine in sich geschlossene Vorsprung (4) die Form eines Logos oder dgl. hat.

20. Vorrichtung nach einem der Ansprüche 10 bis 19,
**dadurch gekennzeichnet, daß** der Ober- (1) und der Unterstempel (3) Bestandteil einer als Rundläufer oder Rotationspresse ausgebildeten Tablettenpresse sind.

21. Vorrichtung nach Anspruch 20,
**dadurch gekennzeichnet, daß** die Dosierstation eine von der Steuerung der Tablettenpresse unabhängige Steuereinheit aufweist, an der die Signale von Näherungssensoren anliegen, so daß die Dosierstation an einer herkömmlichen Tablettenpresse nachrüstbar ist.

22. Vorrichtung nach einem der Ansprüche 10 bis 21,
**dadurch gekennzeichnet, daß** wenigstens eine Station vorgesehen ist, die eine Beschichtung aufbringt.

## Claims

1. Process for the manufacture of a tablet or similar devices comprising at least one coating material (51) and at least one core material (52), which can consist specifically of viscose material, by the use of a tablet press having an upper punch (1) and preferably also a mobile bottom punch (3),
**characterised in that** the bottom punch (3) is provided with at least one recess in the stamp itself or in at least one projection point (4) of the stamp, and
that the core material(s) (52) is/are inserted into the recess(es) before the coating material(s) (51) is/are inserted in the die plate formed by the bottom punch (3) and the side walls (2).

2. Process for the manufacture of a tablet or similar devices comprising at least one coating material (51) and at least one core material, which can consist specifically of viscose material, by the use of a tablet press having an upper punch (1) and preferably also a mobile bottom punch (3),
**characterised in that** the coating material (51) is filled into the die plate formed by the bottom punch (3) and the side walls (2) and that the core material (52) is then placed onto the surface of the coating material (51) facing the upper punch (1), and
that the tablets are then pressed with an upper punch (1) that is provided with at least one recess in the stamp itself or in at least one projection point (4) of the stamp, which limits the spread of the different core material (52) or coating material (51) in the pressed tablet (5).

3. Process for the manufacture of a tablet or similar devices comprising at least one coating material (51) and at least one core material (52), which can consist specifically of viscose material, by the use of a tablet press having an upper punch (1) and preferably also a mobile bottom punch (3),
**characterised in that** the upper punch (1) provides for at least one projection point (4) encompassing an area in which the core material is inserted, and that the coating material (51) is filled into the die plate formed by the bottom punch (3) and the side walls (2).

4. Process in accordance with one of the claims 1 to 3, **characterised in that** the tool in contact with the core material(s) (52), i.e. the bottom punch (3) or the upper punch (1), is cooled by the use of a melting for the core material(s).

5. Process in accordance with one of the claims 1 to 4, **characterised in that** the pressing step takes place after the coating materials) (51) has been filled into the die plate.

6. Process in accordance with one of the claims 1 to 5, **characterised in that** the surface facing the upper punch (1) can be processed into a form after the coating materials) (51) has been filled and prior to the pressing step.

7. Process in accordance with one of the claims 1, 3 to 6, **characterised in that** a separating layer is applied before the core material(s) (52) has been attached to the bottom stamp (3).

8. Process in accordance with one of the claims 2, 4 to 7, **characterised in that** a separating layer has been applied at least onto the core material (52), after the core material(s) (52) has been applied onto the coating material (51) and prior to the pressing step.

9. Process in accordance with one of the claims 1 to 8, **characterised in that** at least one of the core materials (52) is an adhesive material.

10. Process for the manufacture of tablets or similar devices comprising at least one coating material (51) and at least one core material (52), specifically for carrying out the process in accordance with one of the claims 1 to 9, with
- at least one die plate, whose base is preferably at least partially formed by a mobile bottom punch (3)
- an upper punch (1)
- at least one dosing station for each core material (52),
**characterised in that** the upper (1) and/or the bottom punch (3) is provided with at least one recess in the stamp itself or in at least one projection point (4) of the stamp, which encompasses the core material (52) during the pressing operation.

11. Process in accordance with claim 10, **characterised in that** the dosing station(s) feed the core material(s) (52) into the area surrounded by the projection point (4) or into the recess.

12. Process in accordance with claim 10 or claim 11, **characterised in that** the dosing station(s) that feeds the core material (52) to the area of the surface of the coating material(s) (51), which is opposite the area(s) encompassed by the projection point (4) or the recess(es).

13. Process in accordance with one of the claims 10 to 12, **characterised in that** the recess (4) is a constituent part of a second stamp, which can be arranged mobile in the upper (1) or the bottom punch (3).

14. Process in accordance with one of the claims 10 to 13, **characterised in that** the upper punch (1) and/or the bottom punch (3) or the second stamp is provided with a cooling device.

15. Process in accordance with claim 14, **characterised in that** the cooling device is provided with at least one channel through which a cold fluid flows.

16. Process in accordance with one of the claims 10 to 15, **characterised in that** the dosing station has one or more valves for each core material.

17. Process in accordance with claim 16, **characterised in that** the valve(s) are formed in such a way that they can be controlled within a time range of 100 µm proportionally with regard to the approved opening or with regard to the duration of the opening period.

18. Process in accordance with one of the claims 10 to 17, **characterised in that** the dosing station(s) permit removal of the core material(s) (52) with high pressures, so that half rigid smelts can also be applied to the upper punch (1) or the bottom stamp (3).

19. Process in accordance with one of the claims 10 to 18, **characterised in that** at least one projection point (4) closed into itself has the form of a logo.

20. Process in accordance with one of the claims 10 to 19, **characterised in that** the upper punch (1) and the bottom stamp (3) are a constituent part of a tablet press formed as a rotating unit or rotation press.

21. Process in accordance with claim 20, **characterised in that** the dosing station is provided with a control unit independent of the control of the tablet press, on which the signals from proximity sensors are attached, so that the dosing station can be fitted to a conventional tablet press.

22. Process in accordance with one of the claims 10 to 21, **characterised in that** at least one station is provided that applies a coating

## Revendications

1. Procédé pour la fabrication d'un comprimé ou quelque chose de ce genre composé d'au moins une matière enrobante (51) et d'au moins une matière centrale (52), pouvant en particulier être composée de matière visqueuse, en utilisant une presse avec un poinçon supérieur (1) et un poinçon inférieur (3) de préférence également mobile,
**caractérisé par le fait que** le poinçon inférieur (3) est doté d'au moins un creux dans le poinçon lui-même ou dans au moins une portée (4) du poinçon, et
**caractérisé par le fait que** la ou les matières centrales (52) sont introduites dans le ou les creux avant l'introduction de la ou des matières enrobantes (51) dans la matrice formée par le poinçon inférieur (3) et les parois latérales (2).

2. Procédé pour la fabrication d'un comprimé ou quelque chose de ce genre composé d'au moins une matière enrobante (51) et d'au moins une matière centrale (52), pouvant en particulier être composée de matière visqueuse, en utilisant une presse avec un poinçon supérieur (1) et un poinçon inférieur (3) de préférence également mobile,
**caractérisé par le fait que** tout d'abord la matière enrobante (51) est versée dans la matrice formée par le poinçon inférieur (3) et les parois latérales (2), et qu'ensuite la matière centrale (52) est appliquée sur la surface de la matière enrobante (51) tournée vers le poinçon supérieur (1), et
**caractérisé par le fait que** par conséquent le comprimé est pressé à l'aide d'un poinçon supérieur (1) présentant au moins un creux dans le poinçon lui-même ou dans au moins une portée (4) du poinçon limitant la distribution de la matière centrale (52) ou de la matière enrobante (51) correspondante dans le comprimé pressé (5).

3. Procédé pour la fabrication d'un comprimé ou quelque chose de ce genre composé d'au moins une matière enrobante (51) et d'au moins une matière centrale (52), pouvant en particulier être composée de matière visqueuse, en utilisant une presse avec un poinçon supérieur (1) et un poinçon inférieur (3) de préférence également mobile,
**caractérisé par le fait que** le poinçon supérieur (3) prévoit au moins une portée (4) comprenant une zone, dans laquelle la matière centrale est introduite,
et **caractérisé par le fait que** la matière enrobante (51) est versée dans la matrice formée par le poinçon inférieur (3) et les parois latérales (2).

4. Procédé selon une des revendications 1 à 3,
**caractérisé par le fait que** lors de l'utilisation d'une fonte pour la ou les matières centrales (52), l'outil entrant en contact avec le ou les matières centrales, c'est à dire le poinçon inférieur (3) ou supérieur (1) est refroidi.

5. Procédé selon une des revendications 1 à 4,
**caractérisé par le fait que** l'étape de pressage suit le remplissage de la ou des matières enrobantes (51) dans la matrice.

6. Procédé selon une des revendications 1 à 5,
**caractérisé par le fait que** la surface tournée vers le poinçon supérieur (1) est façonnée après le remplissage de la ou des matières enrobantes (51) et avant l'étape de pressage.

7. Procédé selon une des revendications 1,3 à 6,
**caractérisé par le fait qu'**une couche de séparation est appliquée avant l'application de la ou des matières centrales (52) sur le poinçon inférieur (3).

8. Procédé selon une des revendications 2,4 à 7,
**caractérisé par le fait qu'**une couche de séparation est appliquée du moins sur la matière centrale (52) après l'application de la ou des matières centrales (52) sur la matière enrobante (51) et avant l'étape de pressage.

9. Procédé selon une des revendications 1 à 8,
**caractérisé par le fait qu'**au moins une des matières centrales (52) est une matière adhésive.

10. Dispositif pour la fabrication de comprimés ou quelque chose de ce genre composés d'au moins une matière enrobante (51) et d'au moins une matière centrale (52), en particulier pour la réalisation du procédé selon une des revendications 1 à 9, avec
- au moins une matrice dont la partie inférieure est de préférence composée du moins partiellement d'un poinçon inférieur (3) mobile,
- un poinçon supérieur (1) ainsi
- qu'au moins une station de dosage pour chaque matière centrale (52),
**caractérisé par le fait que** le poinçon supérieur (1) et/ou inférieur (3) est doté d'au moins un creux dans le poinçon lui-même ou au moins une portée (4) du poinçon entourant la matière centrale (52) lors de l'étape de pressage.

11. Dispositif selon la revendication 10,
**caractérisé par le fait que** la ou les stations de dosage effectue(nt) un ou des dosages de la ou des matières centrales (52) dans la zone entourée par la portée (4) ou dans le creux.

12. Dispositif selon la revendication 10 ou 11,
**caractérisé par le fait que** la ou les stations de dosage effectue(nt) le dosage de la ou des matières centrales (52) dans la zone de la surface de la ou des matières enrobantes (51) étant située sur le côté opposé à la ou les zones entourée(s) par la portée (4) ou au(x) creu(x).

13. Dispositif selon une des revendications 10 à 12,
**caractérisé par le fait que** le creux fait partie d'un deuxième poinçon disposé de façon mobile dans le poinçon supérieur (1) ou dans le poinçon inférieur (3).

14. Dispositif selon une des revendications 10 à 13,
**caractérisé par le fait que** le poinçon supérieur (1) et/ou le poinçon inférieur (3) ou le deuxième poinçon présentent un dispositif réfrigérant.

15. Dispositif selon la revendication 14,
**caractérisé par le fait que** le dispositif réfrigérant possède au moins un canal par lequel coule un fluide froid.

16. Dispositif selon une des revendications 10 à 15,
**caractérisé par le fait que** la station de dosage possède une ou plusieurs vannes pour chaque matière centrale.

17. Dispositif selon la revendication 16,
**caractérisé par le fait que** la ou les vannes sont configurées de telle sorte, qu'elles sont accessibles dans un laps de temps de 100 µs proportionnellement par rapport à l'ouverture libérée ou par rapport à la durée d'ouverture.

18. Dispositif selon une des revendications 10 à 17,
**caractérisé par le fait que** la ou les stations de dosage permet(tent) la répartition de la ou des matières centrales (52) à l'aide d'une forte pression, de sorte que même les fontes à moitié solidifiées sont applicables sur le poinçon supérieur (1) et/ou inférieur (3).

19. Dispositif selon une des revendications 10 à 18,
**caractérisé par le fait qu'**au moins une portée (4) autonome possède la forme d'un logo ou quelque chose de ce genre.

20. Dispositif selon une des revendications 10 à 19,
**caractérisé par le fait que** le poinçon supérieur (1) et le poinçon inférieur (3) font partie d'une presse à fabriquer des comprimés configurée comme machine à plateaux tournants ou presse rotative.

21. Dispositif selon la revendication 20,
**caractérisé par le fait que** la station de dosage présente un élément de contrôle indépendant du contrôle de la presse à fabriquer des comprimés et auquel adhère les signaux de détecteurs de proximité, de telle sorte que la presse à fabriquer des comprimés traditionnelle peut être équipée ultérieurement d'une station de dosage.

22. Dispositif selon une des revendications 10 à 21,
**caractérisé par le fait qu'**il existe au moins une station appliquant une couche.
